(19) Europäisches Patentamt
European Patent Office
Office européen des brevets

(11) Veröffentlichungsnummer: **0 272 221**
**A2**

(12) # EUROPÄISCHE PATENTANMELDUNG

(21) Anmeldenummer: **87810753.1**

(51) Int. Cl.4: **C07D 213/61** , C07D 409/06

(22) Anmeldetag: **14.12.87**

(30) Priorität: **19.12.86 CH 5096/86**

(43) Veröffentlichungstag der Anmeldung:
**22.06.88 Patentblatt 88/25**

(84) Benannte Vertragsstaaten:
**CH DE FR GB LI**

(71) Anmelder: **CIBA-GEIGY AG**
**Klybeckstrasse 141**
**CH-4002 Basel(CH)**

(72) Erfinder: **Weis, Claus D., Dr.**
**Hangelimattweg 112**
**CH-4148 Pfeffingen(CH)**
Erfinder: **Sutter, Peter**
**Seemättlistrasse 14/2**
**CH-4132 Muttenz(CH)**

(54) **Verfahren zur Herstellung von substituierten Pyridinen.**

(57) Ein Verfahren zur Herstellung von Verbindungen der Formel

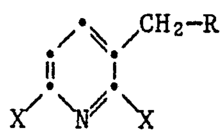

(1),

worin R und X die im Anspruch 1 angegebenen bedeutung haben, wird beschrieben.

Die Verbindungen der Formel (1) sind wertvolle Zwischenprodukte bei der Synthese von Triarylmethanfarbstoffen und Triarylmethanlactonen.

EP 0 272 221 A2

## Verfahren zur Herstellung von substituierten Pyridinen

Die Erfindung betrifft ein Verfahren zur Herstellung von 2,6-Dihalogen-3-arylmethylpyridinen. welche wertvolle Zwischenprodukte bei der Synthese von Triarylmethanfarbstoffen und Triarylmethanlactonen sind.

Es ist bereits, z.B. aus U. Horn, F. Mutterer und C.D. Weis, Helv. Chim. Acta 59, 190ff (1976), ein Verfahren zur Herstellung von 2,6-Dichlor-3-arylmethylpyridinen bekannt, wobei die Arylverbindung mit 2,6-Dichlor-3-chlormethylpyridin gemäss einer Friedel-Crafts-Alkylierungsreaktion umgesetzt wird. Dieses Verfahren birgt jedoch den Nachteil, dass im Fall von substituierten Arylverbindungen im allgemeinen kein einheitliches Produkt, sonderen lediglich ein Gemisch verschiedener stellungsiosomerer Verbindungen erhalten wird, das erst mühsam aufgetrennt werden muss. Ausserdem lassen sich nach dem geschilderten Verfahren nur 2,6-Dihalogenpyridine mit einem carbocyclischen Arylmethylrest, nicht jedoch solche mit einem heterocyclischen Arylmethylrest in 3-Stellung herstellen.

Es wurde nun ein universell anwendbares Verfahren zur Herstellung von 2,6-Dihalogen-3-arylmethylpyridinen gefunden, mit dem sich eine unübersehbare Zahl von carbocyclischen und heterocyclischen Arylmethylsubstituenten in die 3-Position von 2,6-Dihalogenpyridinen einführen lassen.

Die Erfindung betrifft daher ein Verfahren zur Herstellung von Verbindungen der Formel

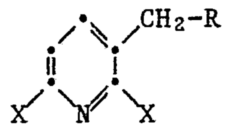

(1),

worin X Halogen und R ein carbocyclischer oder heterocyclischer aromatischer Rest ist, gekennzeichnet durch die Verfahrensschritte:

a) Diazotierung einer Verfindung der Formel

$R-NH_2$ (2)

in Gegenwart eines polaren organischen Lösungsmittels, einer Säure und eines Diazotierungsreagens und Umsetzung der diazotierten Verbindung mit 2-Methylenglutarsäuredinitril in Gegenwart eines Katalysators,

b) Cyclisierung der gemäss a) erhaltenen Verbindung der Formel

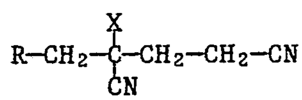

(3)

in saurem Medium zur Piperidin-2,6-dion-Verbindung der Formel

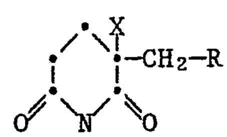

(4)

und

c) Aromatisierung der Piperidin-2,6-dion-Verbindung der Formel (4) in Gegenwart eines dehydratisierenden Lösungsmittels, wobei R und X jeweils die bei der Formel (1) angegebene Bedeutung haben.

In den Verbindungen der Formel (1) bedeutet X Halogen, z.B. Brom und insbesondere Chlor.

R hat in Formel (1) die Bedeutung eines carbocyclischen oder heterocyclischen aromatischen Restes; vorzugsweise handelt es sich dabei um den Rest eines carbocyclischen $C_6$-$C_{18}$-Aromaten oder um den Rest eines mono-oder bicyclischen Heteroaromaten, welcher ein oder mehrere N-, S-und.oder O-Atome aufweist.

Der Rest R kann unsubstituiert oder einfach oder mehrfach durch gleiche oder verschiedene Reste substituiert sein. Geeginete Substituenten sind z.B. $C_1$-$C_4$-Alkyl wie Methyl, Ethyl, n-oder iso-Propyl oder n-, sec.-oder tert-Butyl, $C_1$-$C_4$-Alkoxy wie Methoxy, Ethoxy, n-oder iso-Propoxy oder n-, sec-oder tert.-Butoxy, Halogen wie Fluor, Chlor oder Brom, Nitro, Cyano oder $C_1$-$C_4$-Alkoxycarbonyl wie Methoxycarbonyl, Ethoxycarbonyl, n-oder iso-Propoxycarbonyl oder n-, sec. oder tert.-Butoxycarbonyl.

R hat bevorzugt die Bedeutung eines unsubstituierten oder z.B. mit den oben genannten Resten

substituierten Phenyl-, Naphthyl-, Pyridinyl-oder Thiophenylrestes.

Eine Gruppe von brauchbaren Verbindungen der Formel (1), die sich nach dem erfindungsgemässen Verfahren herstellen lassen, umfasst solche, worin R ein carbocyclischer aromatischer Rest ist; hierbei steht R bevorzugt für eine unsubstituierten oder wie oben geschildert substituierten Naphthyl-und insbesondere Phenylrest. Das Verfahren ist insbesondere geeignet zur Herstellung von Verbindungen der Formel (1), worin R ein unsubstituierter oder durch Methyl, Ethyl, Methoxy, Ethoxy, Chlor, Fluor, Nitro, Cyano, Methoxycarbonyl und/oder Ethoxycarbonyl substituierter Phenylrest ist.

Eine weitere Gruppe von brauchbaren Verbindungen der Formel (1), die mittels des erfindungsgemässen Verfahrens erhalten werden können umfasst solche, worin R ein heterocyclischer aromatischer Rest ist; hierbei stellt R vorzugsweise den Rest eines mono-oder bicyclischen Heterocyclus dar, der ein oder mehrere N-und/oder S-Atome aufweist.

Beispiele für geeignete heterocyclische Reste R sind der Rest des Pyridins oder Thiphens, wobei diese Reste durch die zuvor genannten Substituenten substituiert sein können.

R steht als heterocyclischer Rest besonders bevorzugt für einen unsubstituierten oder durch Methyl, Chlor, Methoxycarbonyl oder Ethoxycarbonyl substituierten Pyridin-3-yl-oder Thiophen-3-yl-Rest.

Bei den erfindungsgemäss in das Verfahren eingestzten Aminen der Formel (2) gelten für R die zuvor genannten Bedeutungen und Bevorzugungen. Demgemäss handelt es sich bei $R\text{-}NH_2$ als carbocyclischem aromatischem Amin bevorzugt um eine unsubstituiertes oder wie zuvor geschildert substituiertes Anilin oder Naphthylamin. Beispiele für bevorzugte heterocyclische aromatische Amine der Formel (2) sind unsubstituiertes oder substituiertes 3-Amino-pyridin oder 3-Amino-thiophen; besonders bevorzugte heterocyclische Amine der Formel (2) sind unsubstituiertes oder durch Methyl, Chlor, Methoxycarbonyl oder Ethoxycarbonyl substituiertes 3-Aminopyridin oder 3-Aminothiophen.

Bei dem polaren organischen Lösungsmittel im Verfahrensschritt a) handelt es sich z.B. um ein Dimethylalkanophosphonat mit 1 bis 4 C-Atomen; weiterhin kommen in Frage z.B. $C_1$-$C_4$-Alkanole wie z.B. Methanol, Ethanol, n-oder iso-Propanol oder n-oder iso-Butanol, $C_1$-$C_4$-Ketone wie z.B. Aceton, $C_4$-$C_{10}$-Glykolether wie Diethylenglykoldimethylether oder -diethylether, Formamide wie Formamid, N-Methylformamid, N,N-Dimethyl-oder N,N-Diethylformamid, oder cyclische Sulfone wie z.B. Sulfolan.

Bevorzugt verwendet man Dimethyl-$C_1$-$C_2$-alkanphosphonate, Methanol, Aceton, Diethylenglykoldimethylether, Diethylenglykoldiethylether, N,N-Dimethylformamid oder Sulfolan als Lösungsmittel im Reaktionsschritt a); das besonders bevorzugte Lösungsmittel ist Dimethylmethanphosphonat.

Das polare organische Lösungsmittel wird z.B. in einem Gewichtsverhältnis von 2:1 bis 10:1, bezogen auf das Amin der Formel (2), eingesetzt.

Als Diazotierungsagentien werden Salpetrigsäureester, insbesondere solche von primären oder sekundären Alkoholen mit 1 bis 10 Kohlenstoffatomen verwendet. Beispiele solcher Ester sind die flüssigen Nitrite von Isopropyl-, Butyl, Pentyl-, Isopentyl-, Heptyl-und Decylalkohol, mit einem Siedepunkt über 50°C; vorteilhaft verwendet man die Ester von Alkoholen mit 1 bis 5 Kohlenstoffatomen wie z.B. Methyl-, Ethyl-, Propyl-, Isopropyl-, Isobutyl-oder Isopentylnitrit, die gasförmig oder flüssig sind. Die Ester können als solche oder in einem Lösungsmittel gelöst, zum Beispiel in dem gleichen, in welchem die Reaktion abläuft, dem Reaktionsgemisch zugeführt werden; die niedrigsiedenden Ester können auch in gasförmigem Zustand eingeleitet werden. Vorzugsweise wird Isopentyl-oder Methylnitrit verwendet.

Das Diazotierungsagens wird vorteilhaft in etwa stöchiometrischen Mengen, bezogen auf das Amin der Formel (2), eingesetzt.

Die Diazotierung wird, wie allgemein üblich, in Gegenwart einer Säure durchgeführt; geeignet Säuren sind z.B. Bromwasserstoffsäure und insbesondere Salzsäure.

Die Säure ist im allgemeinen mindestens in äquimolaren Mengen und vorzugsweise in einem bis zu 5fachen molaren Ueberschuss, bezogen auf das Amin der Formel (2), anwesend.

Die Diazotierung wie auch die Umsetzung der diazotierten Verbindung der Formel (2) mit 2-Methylenglutarsäuredinitril werden z.B. bei einer Temperatur von 20 bis 100°C und vorzugsweise 35 bis 75°C ausgeführt.

Insbesondere bevorzugt ist es, die Diazotierung und die Umsetzung der diazotierten Verbindung mit 2-Methylenglutarsäuredinitril simultan durchzuführen, da dann die diazotierte Verbindung stets nur in geringen Mengen vorhanden ist.

Das 2-Methylenglutarsäuredinitril wird vorteilhaft in einem Molverhältnis von 1:1 bis 5:1 und vorzugsweise 1:1 bis 2:1, bezogen auf das Amin der Formel (2), eingesetzt.

Als Katalysator für die Umsetzung der diazotierten Amine der Formel (2) mit 2-Methylenglutarsäuredinitril kommen z.B. metallisches Eisen-oder Kupferpulver, Eisen-oder Kupfersalze oder aus Eisenpulver und -salz oder Kupferpulver und -salz bestehende Gemische in Frage; bei den Metallsalzen handelt es sich bevorzugt um die entsprechenden Chloride. Wird ein Gemisch aus Metallpulver und -salz

verwendet, so werden beide bevorzugt im Verhältnis 1:1 eingesetzt.

Vorzugsweise benutzt man katalytische Mengen Kupferpulver, Kupfer-(I)-oder Kupfer-(II)-chlorid und insbesondere bevorzugt ist die Verwendung von Kupfer-(I)-chlorid.

Der Katalysator wird in Mengen von z.B. 0,1 bis 5 Gew.-% und vorzugsweise 0,5 bis 2 Gew.-%, bezogen auf das Amin der Formel (2), eingesetzt.

Eine besonders bevorzugte Ausführungsform des Verfahrensschrittes a) des vorliegenden Verfahrens betrifft die Diazotierung eines Amins der Formel (2) in Gegenwart eines Salpetrigsäureesters mit 1 bis 5 C-Atomen, Salzsäure und Dimethylmethanphosphonat, und simultane Umsetzung der diazotierten Verbindungen mit 1 bis 5 Mol 2-Methylenglutarsäuredinitril pro Mol Amin der Formel (2) in Gegenwart von 0,1 bis 5,0 Gew.-%, bezogen auf das Amin der Formel (2), metallischem Kupferpulver, Kupfer-(1)-oder Kupfer-(II)-chlorid bei einer Temperatur von 35 bis 75°C.

Die gemäss Verfahrensschritt a) erhaltenen Dinitrile der Formel (3) lassen sich in saurem Medium bei einer Temperatur von z.B. 40 bis 200°C und vorzugsweise 80 bis 170°C in die Piperidin-2,6-dion-Verbindungen der Formel (4) überführen. Das saure Medium besteht z.B. aus einer anorganischen oder organischen Säure, z.B. Salzsäure, Schwefelsäure, Ameisensäure oder Essigsäure. Geeignet sind auch Gemische verschiedener Säuren und hierbei insbesondere Gemische aus einer anorganischen und einer organischen Säure.

Ein für die Cyclisierung gemäss Verfahrensschritt b) besonders bevorzugtes Medium stellt ein Gemisch aus Essigsäure und Schwefelsäure dar.

Für den Verfahrensschritt c) kommen als wasserentziehende Lösungsmittel z.B. Phosphoroxytribromid und insbesondere Phosphoroxytrichlorid in Frage; das wasserentziehende Lösungsmittel wird jeweils im Ueberschuss, bezogen auf die Verbindung der Formel (4) eingesetzt.

Die Aromatisierung kann ohne Katalysator oder, vorzugsweise, in Anwesenheit eines Katalysators ausgeführt werden; ein geeigneter Katalysator ist z.B. Hexamethylphosphorsäuretriamid (HMPT).

Der Katalysator wird z.B. in einer Menge von 3 bis 30 Gew.-% und vorzugsweise 5 bis 15 Gew.-%, bezogen auf das Dehydratisierungsagens, eingesetzt.

Die Aromatisierung der Piperidin-2,6-dion-Verbindungen der Formel (4) findet z.B. bei einer Temperatur von 50 bis 250°C, vorzugsweise bei 100 bis 200°C, und besonders bevorzugt bei 120 bis 180°C statt.

Die Aromatisierung kann im offenen Gefäss unter Normaldruck oder, vorzugsweise, im abgeschlossenen Gefäss (Autoklav) unter Druck, der z.B. bis zu 10 bar, vorzugsweise bis zu 3 bar, betrágen kann, ausgeführt werden.

In einer bevorzugten Ausführungsform des erfindungsgemässen Verfahrens wird der Aromatisierungsschritt c) in Gegenwart von überschüssigem Phosphoroxytribromid oder Phosphoroxytrichlorid und 5 bis 15 Gew. % HMPT, bezogen auf das Phosphoroxytrihalogenid, im abgeschlossenen Gefäss unter Druck bei einer Temperatur von 120 bis 180°C durchgeführt.

Die Produkte der Verfahrensschritte a), b) und c) können jeweils in an sich bekannter Weise, z.B. durch Filtration, Kristallisation, Extraktion, Waschen und/oder Destillation, isoliert und gereinigt werden.

Eine besonders bevorzugte Ausführungsform des erfindungsgemässen Verfahrens zur Herstellung von Verbindungen der zuvor angegebenen Formel (1) ist gekennzeichnet durch die Verfahrensschritte:

a) Diazotierung einer Verbindung der zuvor angegebenen Formel (2) in Gegenwart eines Salpetrigsäureesters mit 1 bis 5 C-Atomen, Salzsäure und Dimethylmethanphosphonat, und simultane Umsetzung der diazotierten Verbindung mit 1 bis 5 Mol 2-Methylenglutarsäuredinitril pro Mol Amin der Formel (2) in Gegenwart von 0,1 bis 5 Gew.-% Kupferpulver, Kupfer-(I)-oder Kupfer-(II)-chlorid, bezogen auf das Amin der Formel (2), bei einer Temperatur von 35 bis 75°C zur Verbindung der zuvor angegebenen Formel (3);

b) Cyclisierung der Verbindung der zuvor angegebenen Formel (3) in einem Gemisch aus Schwefelsäure und Essigsäure bei einer Temperatur von 40 bis 200°C zur Piperidin-2,6-dion-Verbindung der zuvor angegebenen Formel (4) und

c) Aromatisierung der Verbindung der zuvor angegebenen Formel (4) in Gegenwart von überschüssigem Phosphoroxytrichlorid oder -bromid und 5-15 Gew.-% Hexamethylphosphorsäuretriamid, bezogen auf das Phosphoroxytrihalogenid, im abgeschlossenen Gefäss unter Druck bei einer Temperatur von 120 bis 180°C.

Ein weiterer Gegenstand der Erfindung sind die Verbindungen der zuvor angegebenen Formel (3) sowie das Verfahren zu ihrer Herstellung gemäss Schritt a) des zuvor geschilderten Verfahrens, worin für R, X und die Verfahrensparameter die zuvor genannten Bedeutungen und Bevorzugungen gelten.

Die Erfindung betrifft ausserdem Piperidin-2,6-dione der zuvor angegebenen Formel (4) sowie das Verfahren zu ihrer Herstellung gemäss Schritt b) des zuvor geschilderten Verfahrens, worin für R, X und die

Verfahrensparameter die zuvor genannten Bedeutungen und Bevorzugungen gelten.

Die Verbindungen der Formel (1) sind zum grossen Teil neu. Ein weiterer Gegenstand der Erfindung sind daher Verbindungen der Formel

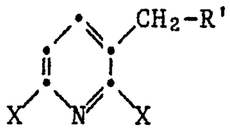

(1a),

worin X Halogen und R' einen heterocyclischen aromatischen Rest oder eine Rest der Formel

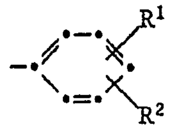

(5)

worin $R^1$ $C_1$-$C_4$-Alkyl, $C_1$-$C_4$-Alkoxy, Halogen, Nitro, Cyano, $C_1$-$C_4$-Alkoxycarbonyl oder Trihalogenmethyl ist und

$R^2$ unabhängig die Bedeutung von $R^1$ hat oder, wenn $R^1$ Cyano, $C_1$-$C_4$-Alkoxycarbonyl oder Trihalogenmethyl ist, auch für Wasserstoff steht, mit den Massgaben, dass einerseits $R^1$ und $R^2$ nicht beide Methyl sind und andererseits $R^1$ bzw. $R^2$ nicht für Nitro steht, wenn der andere Substituent, $R^2$ bzw. $R^1$, Chlor ist, bedeutet.

Bevorzugt sind solche Verbindungen der Formel (1a), worin R' ein Rest der oben angegebenen Formel (5) ist, $R^1$ für Cyano, $C_1$-$C_4$-Alkoxycarbonyl oder Trihalogenmethyl steht und $R^2$ Wasserstoff oder Nitro ist.

Besonders bevorzugt sind Verbindungen der Formel (1a), worin R' eine heterocyclischen aromatischen Rest darstellt; hierbei gelten für R' die zuvor für R als heterocyclischem aromatischem Rest angegebenen Bedeutungen und Bevorzugungen.

Die Verbindungen der Formel (1) bzw. (1a) lassen sich gemäss Schritt c) des zuvor geschilderten Verfahrens aus Piperidin-2,6-dionverbindungen der zuvor angegebenen Formel (4) herstellen; bezüglich der Verbindungen der Formel (4) und der Verfahrensparameter gelten hierbei die zuvor genannten Bedeutungen und Bevorzugungen.

Die Verbindungen der Formeln (1) bzw. (1a) sind wertvolle Zwischenprodukte z.B. bei der Herstellung von Triarylmethanfarbstoffen, die durch Oxidation zum Diarylketon der Formel

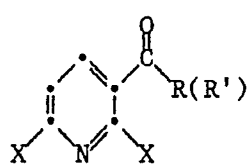

(6)

worin X, R und R' jeweils die zuvor angegebene Bedeutung haben, und Umsetzung des Ketons mit einer weiteren aromatischen Verbindung erhalten werden können.

Weist der aromatische Rest R bzw. R' in den Verbindungen der Formel (6) eine Estergruppe oder eine in eine Estergruppe überführbare funktionelle Gruppe, z.B. Cyano oder Trihalogenmethyl, in ortho-Stellung zur Ketogruppe auf, werden Triarylmethanlactone erhalten, die z.B. als Farbbildner brauchbar sind.

Die Verbindungen der Formel (1) bzw. (1a) ermöglichen somit den Zugang zu einer unübersehbaren Fülle von zum Teil neuen Triarylmethanfarbstoffen und Triarylmethanlactonen; ein weiteres Einsatzgebiet betrifft ihre Verwendung als Zwischenprodukte für Pharmazeutika und Pflanzenschutzmittel.

Die Beispiele verdeutlichen die Erfindung, ohne sie darauf zu beschränken.

Herstellung der Dicyanobutanverbindungen gemäss Verfahrensschritt a)

Beispiel 1:

500 ml Dimethylmethanphosphonat, 93 g Anilin, 152 g konz. Salzsäure, 159 g 2-Methylenglutarsäuredinitril und 0,75 g Kupfer-(1)-chlorid werden zusammen auf 55 - 60°C erhitzt. Das Heizbad wird entfernt und 123,2 g iso-Pentylnitrit tropfenweise unter Rühren addiert (Dauer ca. 1,5 Stunden, abhängig von der Stickstoffentwicklung und dem exothermen Verlauf der Reaktion). Man lässt 1,5 Stunden nachrühren und gibt dann das Reaktionsgemisch auf 2 Liter Wasser; aus der öligen Suspension scheidet sich beim Stehen das feste Produkt ab, welches abgesaugt, mit Wasser gewaschen und aus Methanol umkristallisiert wird.

Es ergeben sich 163 g (85 %) der Verbindung der Formel

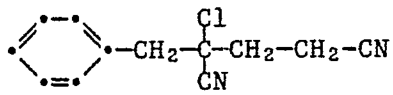 ,

Schmelzpunkt 70 - 71°C.

Beispiel 2:

10,7 g 4-Methylanilin, 50 ml Dimethylmethanphosphonat, 15,2 g konz. Salzsäure und 0,1 g Kupfer-(1)-chlorid werden auf 55 bis 60°C erhitzt und innerhalb von 20 Minuten unter Rühren tropfenweise mit 12,3 g iso-Pentylnitrit versetzt.

Man lässt 30 Minuten nachrühren und gibt das Reaktionsgemisch auf 150 ml Wasser. Anschliessend wird das Produkt mit Ether extrahiert und das Lösungmittel nach dem Trocknen in Vakuum abgedampft. Man kristallisiert das erhaltene Oel aus Methanol und wäscht den Feststoff mit kaltem Methanol. Ausbeute: 15,6 g (86 %) der Verbindung der Formel

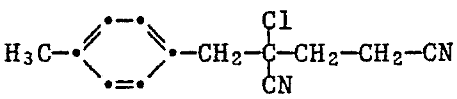,

Schmelzpunkt 68 - 69°C.

Beispiel 3:

Verfährt man wie im Beispiel 1 oder 2 beschrieben und verwendet anstelle von iso-Pentylnitrit die äquivalente Menge Methylnitrit, so erhält man die Verbindungen der Beispiele 1 oder 2 in etwa gleicher Qualität.

Beispiel 4:

Verfährt man wie im Beispiel 1 oder 2 beschrieben, und verwendet anstelle von Dimethylmethanphosphonat die gleiche Gewichtsmenge Ethylenglykoldimethylether, Dimethylformamid oder Sulfolan, werden jeweils die Verbindungen der Beispiele 1 oder 2 in etwa gleicher Qualität erhalten.

Beispiele 5 - 22:

Arbeitet man wie in den Beispielen 1 oder 2 beschrieben und verwendet die in der folgenden Tabelle genannten aromatischen Amine anstatt von Anilin oder 4-Methylanilin, so erhält man analoge Verbindungen mit den in der Tabelle genannten Schmelzpunkten.

| Beispiel | aromatisches Amin R-$NH_2$ | Schmelzpunkt der Verbindung 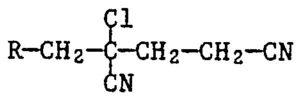 [°C] | Ausbeute [%] |
|---|---|---|---|
| 5 | 4-Methoxyanilin | 55 - 55,5 | 82 |
| 6 | 4-Chloranilin | 68 - 70 | 51 |
| 7 | 4-Fluoranilin | Oel | 58 |
| 8 | 3-Trifluormethylanilin | 50,5 - 51 | 80 |
| 9 | 4-Nitroanilin | 99 - 100 | 84 |
| 10 | 2-Methoxycarbonylanilin | Oel | 60 |
| 11 | 4-Methoxycarbonylanilin | 72 - 73 | 76 |
| 12 | 2,5-Dichloranilin | 68 - 69 | 65 |
| 13 | 3,4-Dichloranilin | 105,5 - 106,5 | 73 |

| Beispiel | aromatisches Amin R-$NH_2$ | Schmelzpunkt der Verbindung 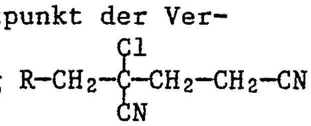 [°C] | Ausbeute [%] |
|---|---|---|---|
| 14 | 2-Methyl-4-nitroanilin | 84 - 85 | 64 |
| 15 | 2-Trifluormethyl-5-nitroanilin | Oel | 38 |
| 16 | 3-Nitro-4-chloranilin | 99 - 100 | 62 |
| 17 | 4-Cyanoanilin | 143 - 144 | 82 |
| 18 | 3-Aminopyridin | 89 - 91 | 43 |
| 19 | 2-Chlor-3-aminopyridin | 109 - 110 | 68 |
| 20 | 2,6-Dichlor-3-aminopyridin | 77 - 78 | 66 |
| 21 | 2,5,6-Trichlor-3-aminopyridin | 112 - 113 | 35 |
| 22 | 2-Methoxycarbonyl-3-aminothiophen | 53,5 - 55 | 60 |

<u>Herstellung der Piperidin-2,6-dion-Verbindungen gemäss Verfahrensschritt b):</u>

<u>Beispiel 23:</u>

6,55 g 1-Phenyl-2-chlor-2,4-dicyanobutan gemäss Beispiel 1, 16,5 ml Essigsäure und 3,7 g 78 % $H_2SO_4$ werden zusammen 2 Stunden unter Rückfluss erhitzt. Man filtriert das Produkt ab, wäscht mit Wasser und erhält 6,25 g der Verbindung der Formel

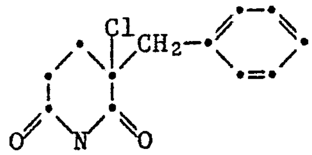

Schmelzpunkt nach Umkristallisation aus Acetonitril 157 - 158°C.

Beispiel 24:

11,0 g 1-(Pyridin-3-yl)-2-chlor-2,4-dicyanobutan gemäss Beispiel 15, 55 ml Essigsäure und 12,5 g 78 % $H_2SO_4$ werden zusammen 15 Stunden unter Rückfluss erhitzt. Das Reaktionsgemisch wird auf ein Gemisch aus Eis und 30 %iger Natriumhydroxidlösung gegeben und anschliessend das Produkt abfiltriert, mit Wasser gewaschen und aus Acetonitril umkristallisiert. Man erhält 8,9 g (75 %) der Verbindung der Formel

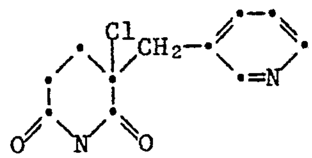

Schmelzpunkt 147 - 148°C.

Beispiele 25 - 43:

Erhitzt man die in der Tabelle aufgeführten 1-Phenyldicyanobutanderivate 2 bis 6 Stunden und die 1-Pyridyldicyanobutanderivate 15 bis 17 Stunden analog zu Beispiel 23 oder 24 in einem Gemisch aus Essigsäure und Schwefelsäure unter Rückfluss, so erhält man analoge Piperidin-2,6-dion-Verbindungen mit den in der Tabelle angegebenen Schmelzpunkten.

| Beispiel | Dicyanobutan-Verbindung 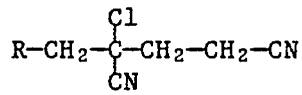 R = | Piperidin-2,6-dion-Verbindung 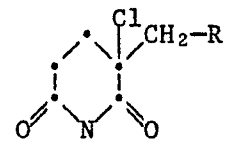 Schmelzpunkt [°C] | Ausbeute [%] |
|---|---|---|---|
| 25 | 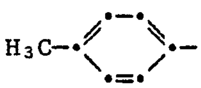 | 197 - 198 | 95 |
| 26 | 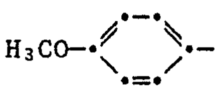 | 140,5 - 141 | 90 |
| 27 | 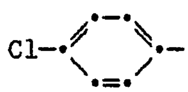 | 161 - 162 | 96 |
| 28 | 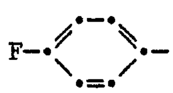 | 171 - 173 | 96 |
| 29 | 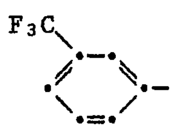 | 127,5 - 129 | 22 |

| Beispiel | Dicyanobutan-Verbindung 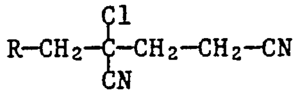 R = | Piperidin-2,6-dion-Verbindung 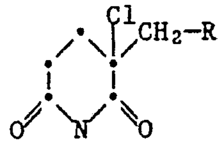 Schmelzpunkt [°C] | Ausbeute [%] |
|---|---|---|---|
| 30 | 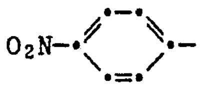 | 191 - 192 | 96 |
| 31 | 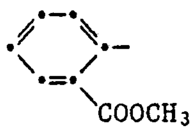 | 113 - 114 | 51 |
| 32 | 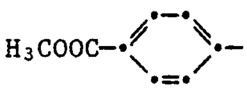 | 172 - 174 | 95 |
| 33 | 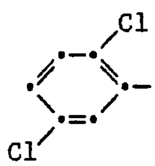 | 199 - 200,5 | 96 |
| 34 | 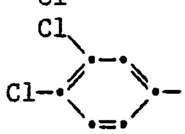 | 151 - 152 | 99 |
| 35 | 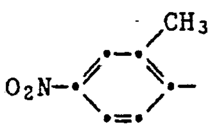 | 228 - 229 | 97 |
| 36 | 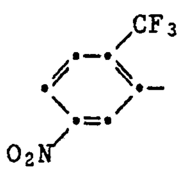 | 185 - 186 | 91 |
| 37 | 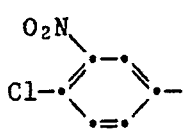 | 173 - 173,5 | 99 |
| 38 | 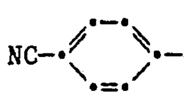 | 163 - 165 | 82 |
| 39 | 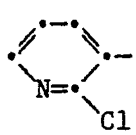 | 147 - 148 | 75 |

| Beispiel | Dicyanobutan-Verbindung 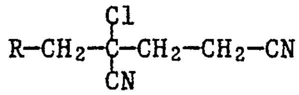 R = | Piperidin-2,6-dion-Verbindung 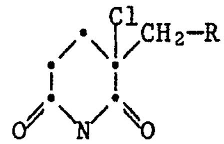 Schmelzpunkt [°C] | Ausbeute [%] |
|---|---|---|---|
| 40 | 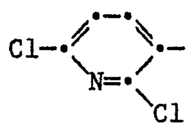 | 189 - 190 | 79 |
| 41 | 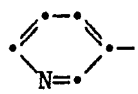 | 147 - 148 | 75 |
| 42 | 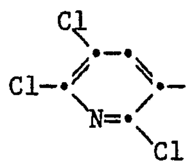 | 217 - 218 | 70 |
| 43 | 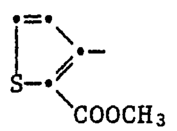 | 146 - 148 | 90 |

Herstellung der 3-Arylmethylpridin-Verbindungen gemäss Verfahrensschritt c):

Beispiel 44:

4,75 g 3-Chlor-3-benzyl-piperidin-2,6-dion gemäss Beispiel 23, 7.6 ml Phosphoroxytrichlorid und 1,0 g Hexamethylphosphorsäuretriamid (HMPT) werden zusammen 7 Stunden unter Rückfluss erhitzt. Nach dem Abkühlen gibt man das Reaktionsgemisch in kaltes Wasser und rührt weitere 20 Minuten. Die wässrige Suspension wird mit Ether extrahiert und die vereinigten Etherextrakte getrocknet. Nach dem Verdampfen des Lösungsmittels im Vakuum fällt ein Oel an, welches bei 118 - 120°C/0,02 mm destilliert wird. Es ergeben sich 2,4 g (50 %) der Verbindung der Formel

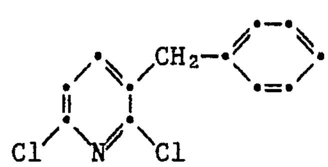

Beispiel 45:

4,08 g 3-Chlor-3-(4-chlorbenzyl)-piperidin-2,6-dion gemäss Beispiel 27 und 22 ml Phosporoxytrichlorid werden im Autoklaven auf eine Temperatur von 170°C erhitzt und 3 Stunden bei dieser Temperatur gehalten. Anschliessend wird das Reaktionsgemisch langsam in 120 ml Wasser gegeben und 20 Minuten gerührt. Das Produkt wird in Ether aufgenommen, die etherische Lösung mit Wasser gewaschen und anschliessend das Lösungsmittel im Vakuum abgedampft. Es ergeben sich 3,1 g (76 %) Verbindugne der Formel

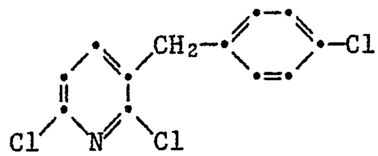

,

Schmelzpunkt 82 - 84°C

Beispiel 46:

56,5 g 3-Chlor-3-(4-nitrophenyl)-piperidin-2,6-dion gemäss Beispiel 30, 300 ml Phosphoroxytrichlorid und 20,5 g HMPT werden 3 Stunden bei 150 - 160°C im Autoklaven erhitzt. Das Reaktionsgemisch wird langsam in 2400 ml Wasser gegeben, und das Ganze 1 Stunde gerührt. Das rohr Produkt wird abfiltriert, mit Wasser gewaschen und in einen Soxhlet überführt, wo es mit Ether extrahiert wird. Nach dem Verdampfen des Lösungsmittels im Vakuum ergeben sich 42,8 g (91 %) der Verbindung der Formel

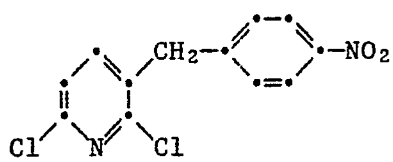

,

Schmelzpunkt 131 - 132°C

Beispiele 47 - 63:

Verfährt man wie im Beispiel 44, 45 oder 46 beschrieben, und verwendet die in der Tabelle angegebenen Piperidin-2,6-dionverbindungen, so erhält man analoge 3-Arylmethylpyridinverbindungen:

| Beispiel | Piperidin-2,6-dion-Verbindung 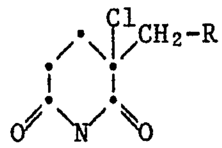 R = | 3-Arylmethylpyridin 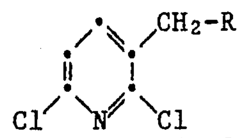 Schmelzpunkt [°C] | Ausbeute [%] |
|---|---|---|---|
| 47 | 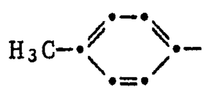 | Oel | 73 |
| 48 | 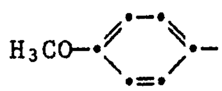 | Oel | 49 |
| 49 | 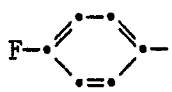 | 73 - 74 | 55 |
| 50 | 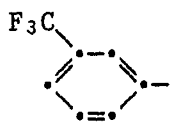 | Oel | 72 |
| 51 | 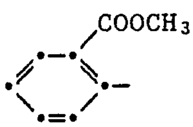 | 69 - 70 | 56 |
| 52 | 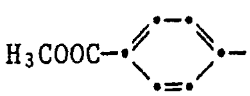 | Oel | 41 |
| 53 | 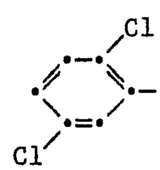 | 57 - 58 | 72 |
| 54 | 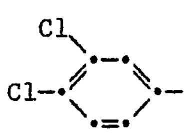 | 94,5 - 96 | 93 |
| 55 | 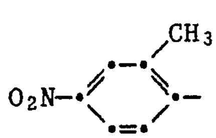 | 118 - 119 | 67 |
| 56 | 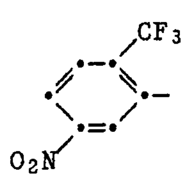 | 79 - 79,5 | 46 |

| Beispiel | Piperidin-2,6-dion-Verbindung | 3-Arylmethylpyridin | Ausbeute [%] |
|---|---|---|---|
| | 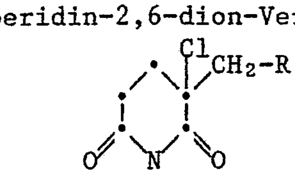 R = | 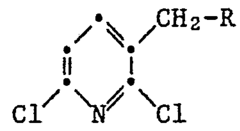 Schmelzpunkt [°C] | |
| 57 | 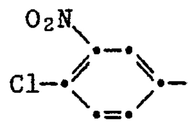 | 91 - 92 | 88 |
| 58 | 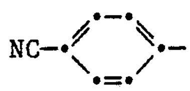 | 87 - 88 | 41 |
| 59 | 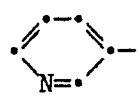 | 55 - 56 | 52 |
| 60 | 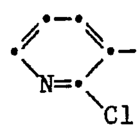 | 108 - 109 | 83 |
| 61 | 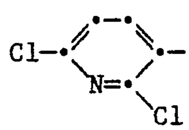 | 142 - 143 | 82 |
| 62 | 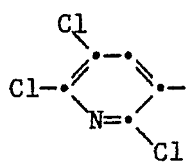 | 107 - 108,5 | 40 |
| 63 | 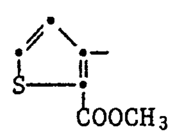 | 103 - 104 | 23 |

**Ansprüche**

1. Verfahren zur Herstellung von Verbindungen der Formel

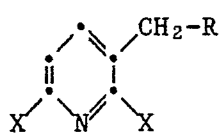

(1),

worin X Halogen und R ein carbocyclischer oder heterocyclischer aromatischer Rest ist, gekennzeichnet durch die Verfahrensschritte:
a) Diazotierung einer Verbindung der Formel
$R\text{-}NH_2$ (2)
in Gegenwart eines polaren organischen Lösungsmittels, einer Säure und eines Diazotierungsreagens und

Umsetzung der diazotierten Verbindung mit 2-Methylenglutarsäuredinitril in Gegenwart eines Katalysators.
b) Cyclisierung der gemäss a) erhaltenen Verbindung der Formel

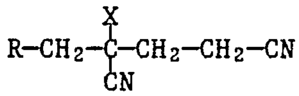 (3)

in saurem Medium zur Piperidin-2,6-dion-Verbindung der Formel

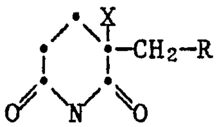 (4)

und
c) Aromatisierung der Piperidin-2,6-dion-Verbindung der Formel (4) in Gegenwart eines dehydratisierenden Lösungsmittels,
wobei R und X jeweils die bei der Formel (1) angegebene Bedeutung haben.

2. Verfahren gemäss Anspruch 1, dadurch gekennzeichnet, dass X für Chlor steht.

3. Verfahren gemäss einem der Ansprüche 1 oder 2, dadurch gekennzeichnet, das R der Rest eines carbocyclischen $C_6$-$C_{18}$-Aromaten oder der Rest eines mono-oder bicyclischen Heteroaromaten, der ein oder mehrere N-, S-und/oder O-Heteroatome aufweist, ist.

4. Verfahren gemäss Anspruch 3, dadurch gekennzeichnet, dass R ein unsubstituierter oder substituierter Phenyl-, Naphthyl-, Pyridinyl-oder Thiophenylrest ist.

5. Verfahren gemäss einem der Ansprüche 1 bis 4, dadurch gekennzeichnet, dass R eine unsubsituierter oder durch Methyl, Ethyl, Methoxy, Ethoxy, Chlor, Fluor, Nitro, Cyano, Methoxycarbonyl, Ethoxycarbonyl und/oder Trifluormethyl substituierter Phenylrest ist.

6. Verfahren gemäss einem der Ansprüche 1 bis 4, dadurch gekennzeichnet, dass R ein unsubstituierter oder durch Methyl, Chlor, Methoxycarbonyl oder Ethoxycarbonyl substituierter Pyridin-3-yl-oder Thiophen-3-yl-Rest ist.

7. Verfahren gemäss einem der Ansprüche 1 bis 6, dadurch gekennzeichnet, dass man als polares organisches Lösungsmittel im Verfahrensschritt a) ein Dimethylalkanphosphonat, $C_1$-$C_4$-Alkanol, $C_1$-$C_4$-Keton, $C_4$-$C_{10}$-Glykolether, unsubstituiertes oder mono-oder di-$C_1$-$C_4$-alkylsubstituiertes Formamid oder ein cyclisches Sulfon verwendet.

Verfahren gemäss Anspruch 7, dadurch gekennzeichnet, dass man ein Dimethyl-$C_1$-$C_2$-alkanphosphonat, Methanol, Aceton, Diethylenglykoldimethylether, Diethylenglykoldiethylether, N,N-Dimethylformamid oder Sulfolan verwendet.

9. Verfahren gemäss einem der Ansprüche 1 bis 8, dadurch gekennzeichnet, dass man Dimethylmethanphosphonat als polares organisches Lösungsmittel verwendet.

10. Verfahren gemäss einem der Ansprüche 1 bis 9, dadurch gekennziechnet, dass man eine Salpetrigsäureester mit 1 bis 5 C-Atomen als Diazotierungsagens verwendet.

11. Verfahren gemäss einem der Ansprüche 1 bis 10, dadurch gekennzeichnet, dass man ein Metall der VIII. oder I. Nebengruppe, ein Salz dieser Metalle oder eine Mischung aus einem entsprechenden Metallsalz und Metallpulver als Katalysator im Verfahrensschritt a) verwendet.

12. Verfahren gemäss Anspruch 11, dadurch gekennzeichnet, dass man 0,1 bis 5 Gew.-%, bezogen auf die Verbindung der Formel (2), Kupfer-(I)-chlorid als Katalysator verwendet.

13. Verfahren gemäss einem der Ansprüche 1 bis 12, dadurch gekennzeichnet, dass man die Diazotierung und Umsetzung mit 2-Methylenglutarsäuredinitril simultan bei einer Temperatur von 35 bis 75°C durchführt.

14. Verfahren gemäss einem der Ansprüche 1 bis 13, dadurch gekennzeichnet, dass man die Cyclisierung gemäss Verfahrensschritt b) in einem Gemisch aus Schwefelsäure und Essigsäure durchführt.

15. Verfahren gemäss einem der Ansprüche 1 bis 14, dadurch gekennzeichnet, dass man Phosphoroxytrichlorid oder -tribromid als dehydratisierendes Lösungsmittel im Reaktionsschritt (c) verwendet.

16. Verfahren gemäss einem der Ansprüche 1 bis 15, dadurch gekennzeichnet, dass man die Aromatisierung gemäss Verfahrensschritt c) in Gegenwart eines Katalysators ausführt.

17. Verfahren gemäss Anspruch 16, dadurch gekennzeichnet, dass es sich bei dem Katalysator um Hexamethylphosphorsäuretriamid handelt.

18. Verfahren gemäss einem der Ansprüche 1 bis 17, dadurch gekennzeichnet, dass man die Aromatisierung gemäss c) im abgeschlossenen Gefäss unter Druck ausführt.

19. Verfahren gemäss einem der Ansprüche 1 bis 18, dadurch gekennzeichnet, dass man die Aromatisierung der Verbindungen der Formel (4) in Gegenwart von Phosphoroxytrichlorid oder -tribromid und Hexamethylphosphorsäuretriamid bei einer Temperatur von 120 bis 180°C unter Druck durchführt.

20. Verfahren zur Hestellung von Verbindungen der Formel (1) gemäss Anspruch 1, gekennzeichnet durch die Verfahrensschritte:

a) Diazotierung einer Verbindung der Formel (2) gemäss Anspruch 1 in Gegenwart eines Salpetrigsäureesters mit 1 bis 5 C-Atomen, Salzsäure und Dimethylmethanphosphonat, und simultane Umsetzung der diazotierten Verbindung mit 1 bis 5 Mol 2-Methylenglutarsäuredinitril pro Mol Amin der Formel (2) in Gegenwart von 0,1 bis 5 Gew.-% Kupferpulver, Kupfer-(I)-oder Kupfer-(II)-chlorid, bezogen auf das Amin der Formel (2), bei einer Temperatur von 35 bis 75°C zur Verbindung der Formel (3) gemäss Anspruch 1.

b) Cyclisierung der Verbindung der Formel (3) gemäss Anspruch 1 in einem Gemisch aus Schwefelsäure und Essigsäure bei einer Temperatur von 40 bis 200°C zur Piperidin-2,6-dion-Verbindung der Formel (4) gemäss Anspruch 1, und

c) Aromatisierung der Verbindung der Formel (4) gemäss Anspruch 1 in Gegenwart von überschüssigem Phosphoroxytrichlorid oder -bromid und 5 - 15 Gew.-% Hexamethylphosphorsäuretriamid, bezogen auf das Phosphoroxytrihalogenid, im abgeschlossenen Gefäss unter Druck bei einer Temperatur von 120 bis 180°C.

21. Verbindungen der Formel

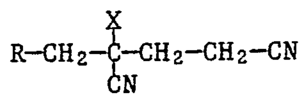 (3),

worin R und X die im Anspruch 1 angegebene Bedeutung haben.

22. Verfahren zur Herstellung von Verbindungen der Formel (3) gemäss Anspruch 21, dadurch gekennzeichnet, dass man eine Verbindung der Formel

$R-NH_2$ (2)

worin R die im Anspruch 1 angegebene Bedeutung hat, in Gegenwart eines polaren organischen Lösungsmittels, einer Säure und eines Diazotierungsreagens diazotiert, und die diazotierte Verbindung mit 2-Methylenglutarsäuredinitril in Gegenwart eines Katalysators umsetzt.

23. Verbindungen der Formel

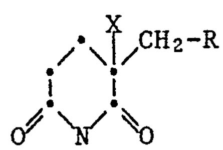 (4),

worin R und X die im Anspruch 1 angegebene Bedeutung haben.

24. Verfahren zur Herstellung von Verbindungen der Formel (4) gemäss Anspruch 23, dadurch gekennzeichnet, dass man eine Verbindung der Formel

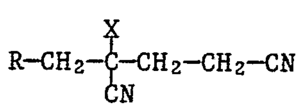 (3),

worin R und X die im Anspruch 1 angegebene Bedeutung haben, in saurem Medium cyclisiert.

25. Verbindung der Formel

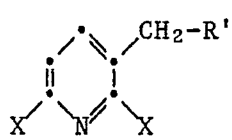 1a),

worin X Halogen und R′ einen heterocyclischen aromatischen Rest oder einen Rest der Formel

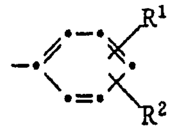

(5),

worin $R^1$ $C_1$-$C_4$-Alkyl, $C_1$-$C_4$-Alkoxy, Halogen, Nitro, Cyano, $C_1$-$C_4$-Alkoxycarbonyl oder Trihalogenmethyl ist und $R^2$ unabhängig die Bedeutung von $R^1$ hat oder, wenn $R^1$ Cyano, $C_1$-$C_4$-Alkoxycarbonyl oder Trihalogenmethyl ist, auch für Wasserstoff steht, mit den Massgaben, dass einerseits $R^1$ und $R^2$ nicht beide Methyl sind und andererseits $R^1$ bzw. $R^2$ nicht für Nitro steht, wenn der andere Substituent, $R^2$ bzw. $R^1$, Chlor ist, bedeutet.

26. Verbindungen gemäss Anspruch 25, worin R′ ein heterocyclischer aromatischer Rest ist.

27. Verbindungen gemäss Anspruch 25, worin R′ ein Rest der im Anspruch 21 angegebenen Formel (5) ist, $R^1$ für Cyano, $C_1$-$C_4$-Alkoxycarbonyl oder Trihalogenmethyl steht und $R^2$ Wasserstoff oder Nitro bedeutet.

28. Verfahren zur Herstellung von Verbindungen der Formel (1) gemäss Anspruch 1, dadurch gekennzeichnet, dass man eine Piperidin-2,6-dion-Verbindung der Formel

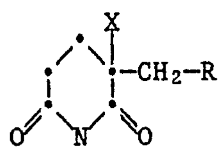

(4),

worin R und X die im Anspruch 1 angegebene Bedeutung haben, in Gegenwart eines dehydratisierenden Lösungsmittels aromatisiert.

29. Verwendung von Verbindungen der Formel (1) gemäss Anspruch 1 als Zwischenprodukte für die Synthese von Triarylmethanfarbstoffen und Triarylmethanlactonen.